# EUROPÄISCHE PATENTSCHRIFT

(11) **EP 0 898 561 B1**
(45) Veröffentlichungstag und Bekanntmachung des Hinweises auf die Patenterteilung: **13.06.2001**
(21) Anmeldenummer: 97921655.3
(22) Anmeldetag: 17.04.1997
(51) Int. Cl.: C07C 409/16

(54) **VERFAHREN ZUR HERSTELLUNG VON 2,5-DIMETHYL-2,5-DI-T-BUTYLPEROXYHEXAN**
PROCESS FOR PREPARING 2,5-DIMETHYL-2,5-DI-T-BUTYLPEROXYHEXAN
PROCEDE DE PREPARATION DE 2,5-DIMETHYL-2,5-DI-T-BUTYLPEROXYHEXANE

(30) Priorität: 17.04.1996 DE 19615182
(43) Veröffentlichungstag der Anmeldung: 03.03.1999
(73) Patentinhaber: Peroxid-Chemie GmbH, 82049 Pullach (DE)
(72) Erfinder: HÄGEL, Eberhard, D-82057 Icking (DE)
(74) Vertreter: Huber, Bernhard, Dipl.-Chem.
(86) Internationale Anmeldenummer: EP9701937
(87) Internationale Veröffentlichungsnummer: WO9738974

(56) Entgegenhaltungen:
- EP-A- 0 262 639
- EP-A- 0 638 551
- GB-A- 954 361
- US-A- 5 210 320

## Beschreibung

Die Erfindung betrifft ein Verfahren zur Herstellung von 2,5-Dimethyl-2,5-di-t-butylperoxy-hexan ausgehend von 2,5 5 Dimethyl-1,5-hexadien und t-Butylhydroperoxid.

2,5-Dimethyl-2,5-di-t-butylperoxy-hexan (DHBP) ist ein wichtiges Peroxid zur Vernetzung von Elastomeren und Thermoplasten und zum gezielten Abbau von hochmolekularem Polypropylen.

Das übliche Herstellverfahren für DHBP geht von 2,5-Dimethyl-hexan-2,5-diol aus. Dieses wird mit Wasserstoffperoxid im stark sauren Medium zum Dihydroperoxid umgesetzt. Durch geeignete Waschschritte wird das überschüssige Wasserstoffperoxid entfernt und das Dihydroperoxid mitt-Butanol in ebenfalls stark saurem Medium zum DHBP weiter umgesetzt.

Nachteile dieses zweistufigen Verfahrens sind die mäßigen Ausbeuten (60 - 65 % bezogen auf eingesetztes Diol), die langen Prozeßzeiten, der Umgang mit dem festen Dihydroperoxid (sicherheitstechnisch kritische Verbindung) und der Anfall von großen Mengen saurer Abwässer und Waschlösungen.

Die Umsetzung von 2,5-Dimethyl-hexan-2,5-diol mit t-Butylhydroperoxid (TBHP) in saurem Medium ist nicht möglich, da das Diol unter Säureeinfluß sehr leicht zum 2,2,5,5-Tetramethyltetrahydrofuran cyclisiert.

Die Umsetzung von 2,5-Dimethyl-1,5-hexadien mit TBHP unter Verwendung eines Säurekatalysators ist in der FR-PS 1 291 965 beschrieben. Als geeignete Säuren sind Perchlorsäure, Schwefelsäure, Chlorwasserstoffgas, Toluolsulfonsäure und ähnlich starke Säuren aufgeführt. Es wird auch erwähnt, daß vorzugsweise in wasserfreiem Medium gearbeitet wird, in den Beispielen wird aber auch 75 prozentiges technisches TBHP eingesetzt. Die Umsetzung von 2,5-Dimethyl-1,5-hexadien mit 75 % TBHP und p-Toluolsulfonsäure führt jedoch nur zu einer Ausbeute von 26 % an DHBP. Aber auch unter wasserfreien Bedingungen lassen sich mit den angegebenen Säuren keine höheren Ausbeuten erzielen. Außerdem ist das in den Beispielen FR-PS verwendete TBHP mit einem Gehalt von 98-99 % eine äußerst gefährliche Substanz, die im technischen Maßstab weder herstellbar noch handhabbar ist. Bei der Umsetzung entstehen außerdem größere Mengen unerwünschter Nebenprodukte, die sich nur durch destillative Reinigung entfernen lassen. Dies ist ein im industriellen Maßstab sicherheitstechnisch nicht vertretbares Verfahren.

Es besteht deshalb ein Bedürfnis an einem Verfahren, welches es gestattet, DHBP sicher und umweltfreundlich mit guten Ausbeuten und in kurzen Prozeßzyklen herzustellen. Der Erfindung liegt die Aufgabe zugrunde, dieses Bedürfnis zu befriedigen.

Überraschend wurde nun gefunden, und hierauf beruht die Erfindung, daß in Gegenwart von Lewis-Säuren in weitgehend wasserfreiem Medium DHBP aus 2,5-Dimethyl-1,5-hexadien und TBHP in guten Ausbeuten und befriedigender Produktqualität in kurzen Reaktionszeiten erhalten werden kann.

Gelöst wird die oben definierte Aufgabe daher erfindungsgemäß durch ein Verfahren zur Herstellung von 2,5-Dimethyl-2,5-di-t-butylperoxy-hexan durch Umsetzung von 2,5-Dimethyl-1,5-hexadien mit t-Butylhydroperoxid unter Säurekatalyse, welches dadurch gekennzeichnet ist, daß man die Umsetzung in Gegenwart einer Elektronenpaarakzeptor-Lewissäure in einem weitgehend wasserfreien Lösungsmittel durchführt.

Vom Sicherheitsstandpunkt her ist es ein wesentlicher Vorteil der Erfindung, daß kein wasserfreies und damit gefährliches TBHP eingesetzt werden muß, sondern daß aus der Mischung von technischem, wasserhaltigem TBHP mit 2,5-Dimethyl-1,5-hexadien das Wasser durch Zugabe von relativ geringen Mengen verdünnter Schwefelsäure oder von Calciumchlorid abgetrennt werden kann. Nach Zugabe des Lewis-Säure-Katalysators erfolgt dann die Umsetzung zum DHBP.

Als geeignete Lewis-Säuren können unter anderen eingesetzt werden:
Bortrifluorid-Etherat, Zinkchlorid-Ether-Komplexe, wasserfreie Komplexe von Schwefelsäure mit Borsäure, Phosphorwolframsäure oder Lithiumperchlorat oder Magnesiumperchlorat in organischen Lösemitteln, wie z.B. Ether.

Die Umsetzung erfolgt vorzugsweise bei einer Temperatur von -10°C bis + 50°C und insbesondere im Bereich von 0°C bis 30°C.

TBHP kann in hochkonzentrierter Form verwendet werden. Bevorzugt wird jedoch technisches wasserhaltiges TBHP mit 60 bis 80 %-iger Konzentration. TBHP wird vorzugsweise in einem molaren Überschuß eingesetzt. Für ein Mol an 2,5-Dimethyl-1,5-hexadien verwendet man besonders bevorzugt 2 bis 6 Mol TBHP, insbesondere 3 bis 4 Mol.

Den Lewis-Säure-Katalysator setzt man bevorzugt in Mengen von 0,1 bis 0,5 Mol pro Mol 2,5-Dimethyl-1,5-hexadien ein, obwohl auch größere oder kleinere Mengen brauchbar sind, abhängig von der jeweils eingesetzten Lewissäure und den Prozeßvariablen.

Das erfindungsgemäße Verfahren vermeidet gefährliche Ausgangsprodukte und liefert mit einfachen Verfahrensmaßnahmen und insbesondere ohne komplizierte oder gefährliche Reinigungsschritte DHBP in Ausbeuten von bis zu 90 % der Theorie mit einer Reinheit bis über 80 %.

### Beispiel 1

Zu einer Mischung aus 170 g (1,5 Mol) 2,5-Dimethyl-1,5-hexadien und 600 g (5,25 Mol) 78 % TBHP gibt man unter Rühren und Kühlen 100 g 72 % Schwefelsäure, rührt 5 Minuten und trennt die wäßrige Phase (185 g) ab. Man gibt unter Rühren und Kühlen nochmals 35 g 72 % Schwefelsäure zu, rührt 5 Minuten und trennt weitere 47 g wäßrige Phase ab.

Unter Rühren und Kühlen tropft man in ca. 35 Minuten eine Lösung von 70 g (0,5 Mol) Bortrifluorid-Diethyletherat in 50 ml Ethylacetat zu, wobei man die Temperatur auf 30°C steigen läßt und rührt noch 3 Stunden bei 30°C nach.

Nach Zugabe von 400 ml Wasser trennt man die wäßrige Phase ab. Das Produkt wird zweimal mit 15 % Natronlauge und zweimal mit Wasser gewaschen, mit wasserfreiem Natriumsulfat getrocknet und filtriert. Nach Entfernen der flüchtigen Bestandteile durch Ausblasen mit Luft bei 50°C erhält man 282 g Produkt mit einem Gehalt an DHBP von 83 %, entsprechend einer Ausbeute von 53,7 % der Theorie .

### Beispiel 2

Man verfährt wie in Beispiel 1, nur gibt man als Katalysator 140 ml (0,3 Mol) einer 2,2-molaren Lösung des Zinkchlorid-Diethylether-Komplexes in Methylenchlorid zu und rührt 2,5 Stunden bei 40°C. Nach Aufarbeiten wie in Beispiel 1 erhält man 308,6 g Produkt mit einem Gehalt an DHBP von 87 %, entsprechend einer Ausbeute von 61,6 % der Theorie.

### Beispiel 3

Man verfährt wie in Beispiel 1, nur gibt man als Katalysator 50 ml (0,25 Mol) einer 5-molaren Lösung von Lithiumperchlorat in Diethylether zu und rührt 2 Stunden bei 30°C. Nach Aufarbeiten wie in Beispiel 1 erhält man 351 g Produkt mit einem Gehalt an DHBP von 91,7 %, entsprechend einer Ausbeute von 73,9 % der Theorie.

### Beispiel 4

Zu einer Mischung aus 85 g (0,75 Mol) 2,5-Dimethyl-1,5-hexadien und 260 g (2,25 Mol) 78 % - TBHP gibt man unter Rühren und Kühlen 45 g 72 % Schwefelsäure, rührt 5 Minuten und trennt die wäßrige Phase (82 g) ab. Man gibt unter Rühren und Kühlen nochmals 15 g 72 % Schwefelsäure zu, rührt 5 Minuten und trennt weitere 22 g wäßrige Phase ab.

Unter Rühren und Kühlen tropft man in ca. 1 Stunde eine Lösung von 22,3 g (0,1 Mol) wasserfreiem Magnesiumperchlorat in 40 g Ethylacetat zu, wobei die Temperatur unter 20°C gehalten wird. Bei 10 bis 12°C rührt man noch 2 Stunden, gibt dann 300 ml Wasser zu und trennt die wäßrige Phase ab. Die organische Phase wird zweimal mit 15 % Natronlauge und zweimal mit Wasser gewaschen. Nach Entfernen der flüchtigen Bestandteile durch Ausblasen mit Luft bei 50°C erhält man 186 g Produkt mit einem Gehalt an DHBP von 94 %, entsprechend einer Ausbeute von 80 % der Theorie.

## Patentansprüche

1. Verfahren zur Herstellung von 2,5-Dimethyl-2,5-di-t-butylperoxy-hexan durch Umsetzung von 2,5-Dimethyl-1,5-hexadien mitt-Butylhydroperoxid unter Säurekatalyse,
**dadurch gekennzeichnet**,
daß man die Umsetzung in Gegenwart einer Elektronenpaarakzeptor-Lewissäure in einem weitgehend wasserfreien Lösungsmittel durchführt.

2. Verfahren nach Anspruch 1,
**dadurch gekennzeichnet**,
daß man als Elektronenpaarakzeptor-Lewissäure mindestens eine Substanz aus der Gruppe Bortrifluoridetherat, Zinkchlorid-Ether-Komplex, wasserfreier Schwefelsäure-Borsäure-Komplex und Lithium- oder Magnesiumperchlorat in organischen Lösemitteln verwendet.

3. Verfahren nach Anspruch 1 oder 2,
**dadurch gekennzeichnet**,
daß man die Elektronenpaarakzeptor-Lewissäure in einer Menge von 0,1 bis 0,5 Mol/Mol 2,5-Dimethyl-1,5-hexadien einsetzt.

4. Verfahren nach einem der vorhergehenden Ansprüche,
**dadurch gekennzeichnet**,
daß man 2 bis 6 Mol t-Butylhydroperoxid pro Mol 2,5-Dimethyl-1,5-hexadien einsetzt.

5. Verfahren nach Anspruch 4,
**dadurch gekennzeichnet**,
daß man 3 bis 4 Mol t-Butylhydroperoxid pro Mol 2,5-Dimethyl-1,5-hexadien einsetzt.

6. Verfahren nach einem der vorhergehenden Ansprüche,
**dadurch gekennzeichnet**,
daß man die Umsetzung bei einer Temperatur von -10 bis + 50°C durchführt.

7. Verfahren nach einem der vorhergehenden Ansprüche,
**dadurch gekennzeichnet**,
daß man technisches wasserhaltiges t-Butylhydroperoxid einsetzt und nach der Mischung mit dem 2,5-Dimethyl-1 ,5-hexadien durch Zugabe von Schwefelsäure oder Calciumchlorid das Wasser abtrennt und anschließend die Elektronenpaarakzeptor-Lewissäure zusetzt.

8. Verfahren nach einem der vorhergehenden Ansprüche,
**dadurch gekennzeichnet**,
daß man nach Beendigung der Umsetzung die Produktphase mit Wasser wäscht.

## Claims

1. Method for the preparation of 2,5-dimethyl-2,5-di-t-butylperoxy-hexane by conversion of 2,5-dimethyl-1,5-hexadiene with t-butyl hydroperoxide under acid catalysis, characterised in that the conversion is carried out in the presence of an electron pair acceptor Lewis acid in a substantially anhydrous solvent.

2. Method according to Claim 1, characterised in that as electron pair acceptor Lewis acid there is used at least one substance from the group boron trifluoride etherate, zinc chloride-ether complex, anhydrous sulphuric acid-boric acid complex and lithium or magnesium perchlorate in organic solvents.

3. Method according to Claim 1 or 2, characterised in that the electron pair acceptor Lewis acid is used in a quantity of from 0.1 to 0.5 mole/mole of 2,5-dimethyl-1,5-hexadiene.

4. Method according to any one of the preceding Claims, characterised in that 2 to 6 moles of t-butyl hydroperoxide per mole of 2,5-dimethyl-1,5-hexadiene are used.

5. Method according to Claim 4, characterised in that 3 to 4 moles of t-butyl hydroperoxide per mole of 2,5-dimethyl-1,5-hexadiene are used.

6. Method according to any one of the preceding Claims, characterised in that the conversion is carried out at a temperature of from -10 to +50°C.

7. Method according to any one of the preceding Claims, characterised in that industrial hydrous t-butyl hydroperoxide is used and, after being mixed with the 2,5-dimethyl-1,5-hexadiene, the water is separated off by adding sulphuric acid or calcium chloride and, subsequently, the electron pair acceptor Lewis acid is added.

8. Method according to any one of the preceding Claims, characterised in that, upon concluding the conversion, the product phase is washed with water.

## Revendications

1. Procédé de préparation du 2,5-diméthyl-2,5-di-t-butylperoxyhexane par réaction du 2,5-diméthyl-1,5-hexadiène avec l'hydroperoxyde de t-butyle en catalyse acide, caractérisé en ce que l'on conduit la réaction en présence d'un acide de Lewis accepteur de doublet électronique dans un solvant sensiblement anhydre.

2. Procédé selon la revendication 1 caractérisé en ce que l'on utilise comme acide de Lewis accepteur de doublet électronique au moins une substance du groupe éthérate de trifluorure de bore, complexe chlorure de zinc-éther, complexe acide sulfurique-acide borique anhydre et perchlorate de lithium ou de magnésium dans des solvants organiques.

3. Procédé selon la revendication 1 ou 2 caractérisé en ce que l'on utilise l'acide de Lewis accepteur de doublet électronique en une quantité de 0,1 à 0,5 mole/mole de 2,5-diméthyl-1,5-hexadiène.

4. Procédé selon l'une des revendications précédentes caractérisé en ce que l'on utilise 2 à 6 moles d'hydroperoxyde de t-butyle par mole de 2,5-diméthyl-1,5-hexadiène.

5. Procédé selon la revendication 4 caractérisé en ce que l'on utilise 3 à 4 moles d'hydroperoxyde de t-butyle par mole de 2,5-diméthyl-1,5-hexadiène.

6. Procédé selon l'une des revendications précédentes caractérisé en ce que l'on conduit la réaction à une température de -10 à +50°C.

7. Procédé selon l'une des revendications précédentes caractérisé en ce que l'on utilise de l'hydroperoxyde de t-butyle hydraté technique et on sépare l'eau après le mélange avec le 2,5-diméthyl-1,5-hexadiène par addition d'acide sulfurique ou de chlorure de calcium, puis on ajoute l'acide de Lewis accepteur de doublet électronique.

8. Procédé selon l'une des revendications précédentes caractérisé en ce qu'on lave à l'eau la phase de produit après la fin de la réaction.
